Europäisches Patentamt

European Patent Office

Office européen des brevets

(11) Numéro de publication : **0 318 385 B1**

(12) **FASCICULE DE BREVET EUROPEEN**

(45) Date de publication du fascicule du brevet :
22.04.92 Bulletin 92/17

(51) Int. Cl.⁵ : **A61M 37/00**

(21) Numéro de dépôt : **88402955.4**

(22) Date de dépôt : **24.11.88**

(54) **Dispositif thérapeutique transdermique et son procédé de préparation.**

(30) Priorité : **27.11.87 FR 8716491**

(43) Date de publication de la demande :
**31.05.89 Bulletin 89/22**

(45) Mention de la délivrance du brevet :
**22.04.92 Bulletin 92/17**

(84) Etats contractants désignés :
**AT BE CH DE ES FR GB GR IT LI LU NL SE**

(56) Documents cités :
**EP-A- 0 072 258**
**FR-A- 2 299 853**
**GB-A- 1 361 289**
**GB-A- 2 093 694**
**US-A- 4 666 441**

(73) Titulaire : **PIERRE FABRE MEDICAMENT**
**45, Place Abel Gance**
**F-92100 Boulogne (FR)**

(72) Inventeur : **Bougaret, Joel**
**36, Boulevard du Maréchal Joffre**
**F-81100 Castres (FR)**
Inventeur : **Sournac, Michel**
**32, rue de Viviers Saix**
**F-81090 Castres (FR)**

(74) Mandataire : **Ahner, Francis et al**
**CABINET REGIMBEAU, 26, avenue Kléber**
**F-75116 Paris (FR)**

EP 0 318 385 B1

## Description

La présente invention concerne un dispositif thérapeutique multilamellaire pour l'administration transdermique de nitroglycérine.

Il existe de nombreux brevets relatifs à des bandages, pièces ou compresses à appliquer sur la peau, pour l'administration de médicaments par voie transdermique vers la circulation générale.

Mais parmi les systèmes transdermiques connus, rares sont ceux qui permettent une cinétique de libération contrôlée en évitant, par exemple un pic de libération important en début de cinétique (phénomène flash).

La présente invention concerne un dispositif thérapeutique multilamellaire pour l'administration transdermique de nitroglycérine comportant successivement :

a) un support réalisé en un matériau souple et occlusif,

b) un réservoir adhésif actif à base de polymères de type acrylique ou silicone, incorporant le principe actif,

c) une membrane de transfert du principe actif, de type polyuréthane hautement perméable à la vapeur d'eau, présentant une structure microporeuse ou continue,

d) une interface adhésive hypoallergénique auto-alimentée par ledit réservoir en principe actif,

e) un protecteur réalisé en un film rigide ou semi-rigide, revêtu d'un anti-adhésif.

Un tel type de dispositif se trouve par exemple décrit dans le brevet GB 1 361 289. Le dispositif selon l'invention est constitué à partir d'un premier module comprenant le support, le réservoir adhésif actif et un papier de protection temporaire et d'un deuxième module comportant la membrane de transfert, l'interface adhésive et le protecteur anti-adhérent, les deux module étant collés l'un sur l'autre après l'enlèvement du papier de protection du premier module par pressage du réservoir adhésif actif contre la membrane perméable du transfert du principe actif.

Le dispositif de la présente invention est tout particulièrement destiné au traitement de fond de l'angine de poitrine.

Les différents strates composant le dispositif de la présente invention possèdent de préférence les structures suivantes.

Le support, souple et occlusif, peut être un film adéquat quelconque tel qu'un complexe aluminium/polyester/polyéthylène mais on choisit de préférence un film thermoplastique coextrudé du type EVA/PVDC/EVA (éthylène vinylacétate/chlorure de polyvinylidène/éthylène vinylacétate).

Dans tous les cas, il est préférable de traiter préalablement la face intérieure de ce support, à savoir la face destinée à adhérer au réservoir adhésif actif. Ce traitement est destiné à favoriser cette adhésion. Il peut s'agir notamment du traitement CORONA.

Ce support peut être teinté dans la masse ou sur sa face extérieure, à savoir la face visible du dispositif de l'invention lorsqu'il est appliqué sur la peau. On choisit de préférence la couleur chair pour que, une fois mis en place, le dispositif selon l'invention soit le plus discret possible.

L'épaisseur du support est, de préférence, comprise entre 50 et 200 μm.

Le réservoir adhésif actif est à base de polymères auxquels on a mélangé intimement le principe actif de préférence sous forme pure, puis que l'on a réticulés.

Dans le cas où le principe actif est la nitroglycérine, on obtient ainsi un film adhésif nitré. Les polymères utilisés sont, de préférence, des polymères de type acrylique ou silicone, notamment utilisables sous forme de solutions ou de dispersions.

Le grammage du principe actif par rapport aux polymères adhésifs réticulés et séchés formant le réservoir adhésif actif est compris entre 0,5 et 10 mg/cm$^2$, et est de préférence égal à environ 2,5 mg/cm$^2$.

Le rapport principe actif/polymères de type acrylique ou silicone compris entre 0,1 et 0,6, de préférence entre 0,2 et 0,3.

L'épaisseur du réservoir adhésif actif est, de préférence, comprise entre 50 et 150 μm.

Divers adjuvants peuvent être incorporés à ce réservoir et/ou à l'interface et notamment des adjuvants de perméation qui favorisent l'absorption du principe actif.

On peut aussi incorporer des agents antiseptiques afin d'éviter toute irritation cutanée due à une prolifération de la flore bactérienne sous de tels dispositifs occlusifs maintenus en place pendant plusieurs jours.

La membrane peut avoir une structure microporeuse ou continue; elle est dans ce dernier cas hautement perméable aux gaz et à la vapeur d'eau et permet la diffusion moléculaire du principe actif vers l'interface adhésive.

La membrane est, de préférence, constituée par un film de polyuréthane.

L'épaisseur de la membrane est, de préférence, comprise entre 10 et 50 μm.

Le transfert progressif de principe actif au travers de cette membrane permet de charger indirectement l'interface adhésive en principe actif. L'interface sera alors "activée". L'auto-alimentation de l'interface par le réservoir en principe actif peut se dérouler, soit au cours d'une phase de maturation lors de la fabrication, soit au cours d'un processus de réalimentation de l'interface lors de l'utilisation.

L'interface adhésive hypoallergénique est de préférence à base de polymères de type acrylique ou silicone. Sa formulation permet d'optimiser les paramètres d'adhésivité tels que pelabilité ou résistance au fluage.

Ainsi, par exemple, le grammage des dérivés constitutifs de l'interface est de préférence compris entre 20 et 60 g/cm$^2$, de préférence entre 30 et 40 g/m$^2$ (exprimé en produit sec).

L'épaisseur de l'interface adhésive est comprise entre 20 et 60 µm, de préférence entre 30 et 40 µm.

La saturation progressive en principe actif de cette interface adhésive va permettre, lors de son application sur la peau, une absorption prolongée du principe actif au travers du stratum cornéeum via le derme et la circulation systémique.

Cette interface adhésive peut, en outre, être chargée d'un agent antiseptique.

Enfin, le protecteur du dispositif transdermique de la présente invention, destiné à être enlevé lors de l'utilisation, est de préférence réalisé en un film choisi parmi les papiers simples ou aluminisés, les polyesters ou les PVC, ou divers complexes stratifiés. L'épaisseur du protecteur est, de préférence, comprise entre 50 et 500 µm.

Ce protecteur est revêtu d'un anti-adhésif choisi parmi les dérivés de silicone, les dérivés fluorés ou de tout autre agent anti-adhésif.

La présente invention concerne aussi le procédé de préparation du dispositif multilamellaire décrit ci-dessus, comportant les étapes consistant à :

a) préparer un premier module comportant successivement :

    . le support,

    . le réservoir adhésif actif,

    . un papier de protection temporaire,

b) préparer un second module comportant successivement :

    . la membrane de transfert du principe actif,

    . l'interface adhésive,

    . le protecteur anti-adhérent,

c) retirer le papier de transfert du premier module puis contre-coller les premier et second modules par pressage du réservoir adhésif actif du premier module contre la membrane perméable au principe actif du second module, et éventuellement

d) laisser reposer les modules contre-collés pour permettre à l'interface adhésive de se saturer progressivement en principe actif.

Selon une variante de ce procédé, on utilise une interface adhésive préalablement chargée en nitroglycérine.

De préférence, l'étape d) est réalisée de la façon suivante : on bobine les modules contre-collés sous forme de rouleaux en vue du stockage.

La présente invention sera mieux comprise à la lecture des exemples et des résultats suivants qui décrivent la préparation d'un dispositif multilamellaire pour la délivrance de nitroglycérine. Ces exemples et résultats sont illustrés par les figures suivantes :

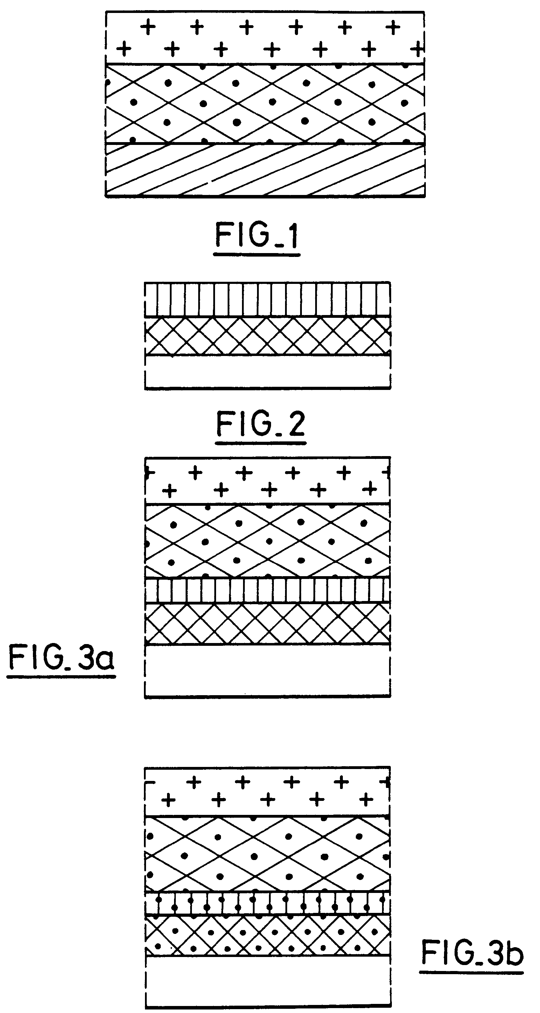

La figure 1 représente le premier module.

Sur cette figure :

représente le support

représente le réservoir adhésif actif

représente le papier de transfert La figure 2 représente le second module.

Sur cette figure :

représente la membrane de polyuréthane

représente l'interface adhésive

représente le protecteur anti-adhérent

La figure 3 représente le dispositif de la présente invention :

3 a - avant stockage,

3 b - après stockage.

Sur cette figure :

EP 0 318 385 B1

représente le support

représente le réservoir adhésif actif

représente la membrane de polyuréthane

représente l'interface adhésive

représente le protecteur anti-adhérent

représente la membrane de polyuréthane chargée en nitroglycérine après le stockage

représente l'interface adhésive activée après le stockage.

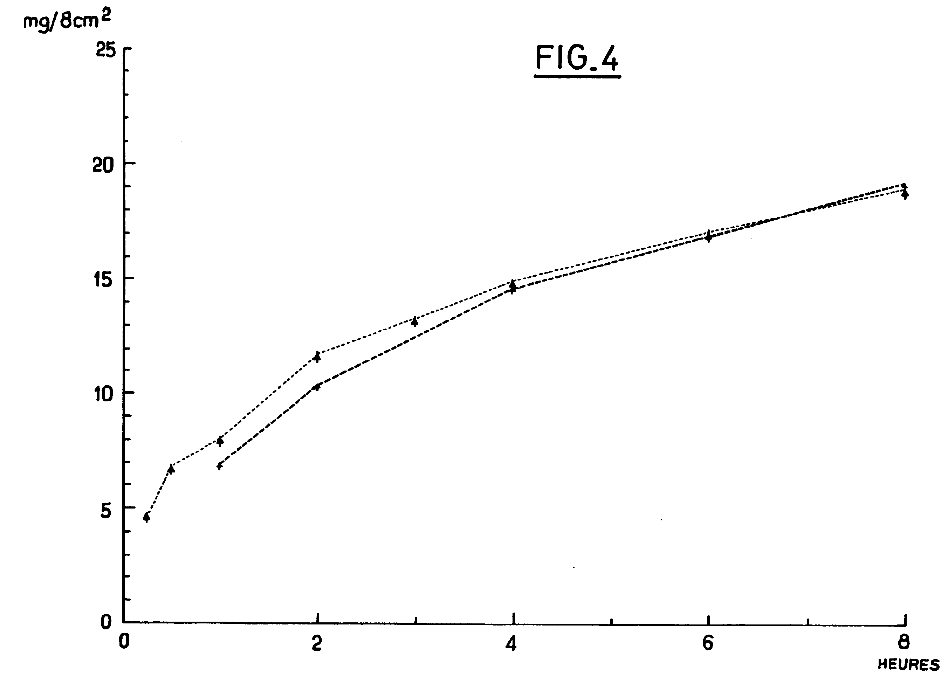

La figure 4 représente la quantité de nitroglycérine libérée (mg/8 cm²) en fonction du temps (heures).

+---+---+ concerne le dispositif au temps zéro, soit après 24 heures de stockage (To)

Δ---Δ---Δ concerne le dispositif à To + 5 mois.

Cet essai a été réalisé dans les conditions suivantes

– dissolutest

– palettes 100 RPM

– eau distillée 900 ml

– 2,5 g/cm² de nitroglycérine dans le réservoir adhésif

– DB5/LOT P100/F01.

La figure 5 représente la quantité de nitroglycérine libérée en fonction du temps (mg/8 cm²) dans les mêmes conditions que pour la figure 4.

Sur cette figure :

_____ représente l'essai réalisé sans membrane (premier module)

_._._. représente l'essai réalisé avec membrane (dispositif de la présente invention)

La figure 6 représente la quantité de nitroglycérine libérée en fonction du temps (mg/8 cm²).

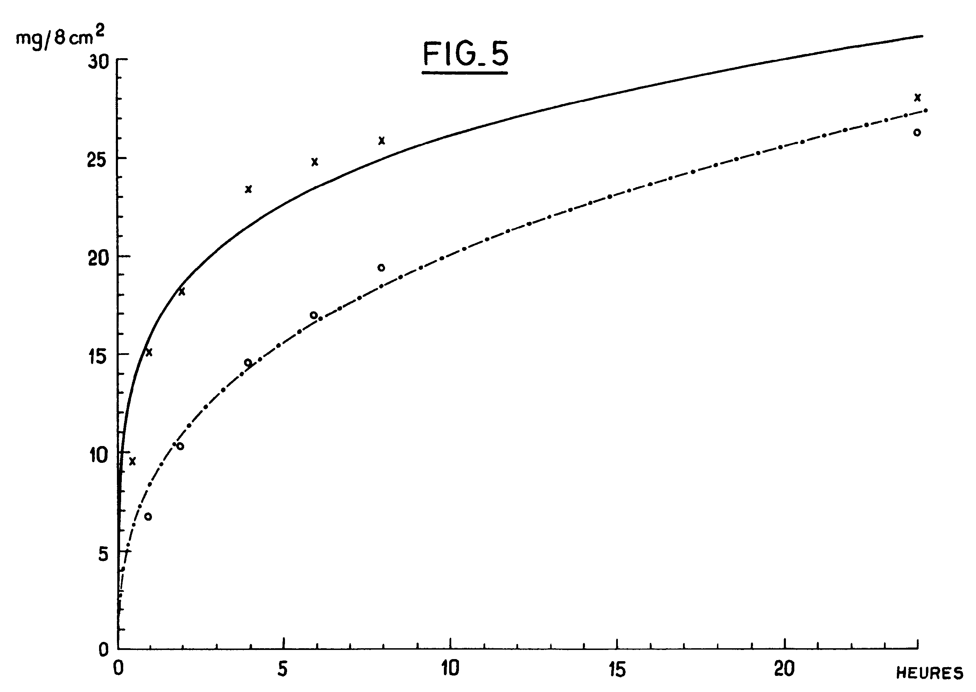

Cet essai a été réalisé pendant 15 minutes dans les mêmes conditions que pour les figures 4 et 5.

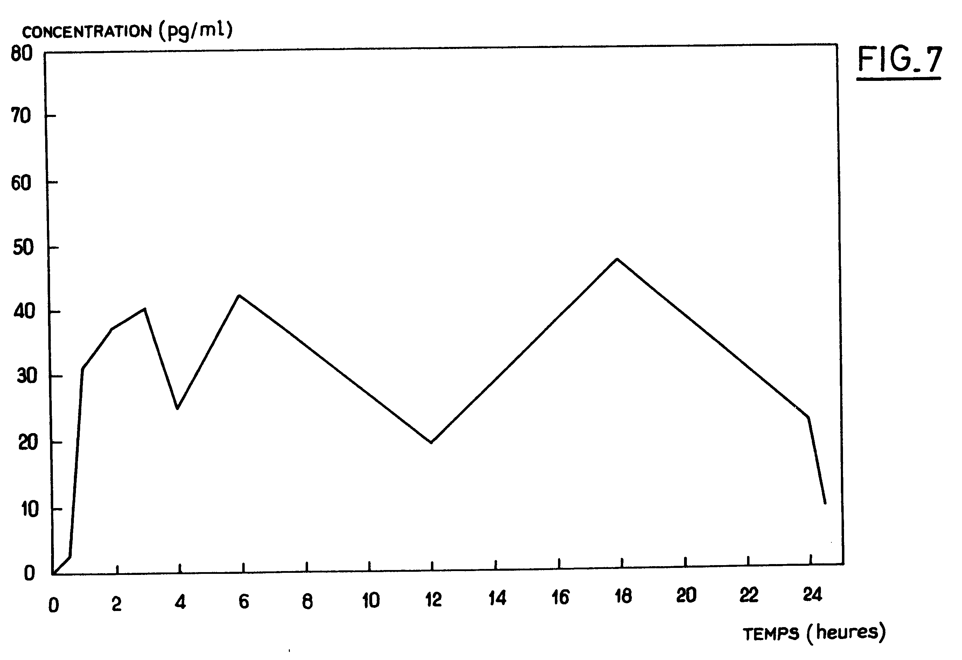

La figure 7 représente les taux plasmatiques moyens en nitroglycérine obtenus après traitement de trois sujets (concentration en pg/ml en fonction du temps en heures).


<u>EXEMPLE</u>


Incorporation de la nitroglycérine au mélange adhésif


On prend comme exemple d'adhésif un polymère du type HG 35 ou HG 37, c'est-à-dire un copolymère acrylique en solution dans un cosolvant à base d'acétate d'éthyle et d'essence A5N.

Partant d'un extrait solide de 40 g/100 g, on charge le produit de base respectivement à 12,5 et 16,7 g/100 g de mélange final, soit la composition suivante :


```
ex 1 : 12.5 %                              (g)

. extrait solide adhésif :                  35

. cosolvant               :                 52,5

. nitroglycérine          :                 12,5

                                        100 g soit 47,5

                                        d'extrait solide

                                        total
```

4

```
ex 2 : 16.7 %                              (g)
. extrait solide adhésif :                 33,3
. cosolvant            :                   50
. nitroglycérine       :                   16,7
                                           100 g soit 50 %
                                           d'extrait solide
                                           total
```

L'incorporation de la nitroglycérine se fait soit directement à l'état pur soit, pour un problème de sécurité et de manutention, sous forme d'une solution fortement concentrée dans l'acétate d'éthyle (afin de limiter le coefficient de dilution et donc la quantité de solvant à évaporer lors du séchage).

Enduction du mélange adhésif actif

L'enduction se fait par exemple sous forme d'un rouleau "lécheur" selon la technique du reverse roll.

La masse adhésive enduite est exprimée en g/m² en entrée machine (grammage exprimé en résine liquide) et en sortie du tunnel de séchage (grammage exprimé en sec).

Ainsi un grammage de 200 g/m² de résine liquide chargée à 12,5 % en nitroglycérine conduira à une résidu sec en sortie machine, après évaporation du solvant de 95 g/m² soit 25 g/m² de nitroglycérine équivalent à 2,5 mg de nitroglycérine/cm².

Un grammage de 200 g/m² de résine liquide chargée à 16,7 % de nitroglycérine conduira à un résidue sec en sortie machine de 100 g/m² soit 33,4 g/m² de nitroglycérine équivalent à 3,34 mg de nitroglycérine/cm².

L'enduction du mélange adhésif actif est effectuée sur un papier intermédiaire anti-adhésif dit papier de transfert.

Séchage et laminage du support

En sortie de la tête d'enduction, le protecteur enduit d'adhésif actif passe dans le tunnel de séchage dont les paramètres (gradient de température, temps de séchage) ont été programmés de façon adéquate afin d'éviter un départ trop important de nitroglycérine tout en limitant le résidu de solvant dans l'adhésif.

L'opération de laminage consiste à contrecoller le film adhésif actif au support traité ou non CORONA (facilite l'accrochage de l'adhésif) par laminage entre des cylindres presseurs.

A ce stade, on obtient le premier module représenté par un schéma sur la figure 1.

Dans ce premier module, l'épaisseur du support est comprise entre 70 et 80 µm et le réservoir adhésif actif mesure environ 100 µm d'épaisseur.

Ce premier module est enroulé en bobines qui seront reprises ci-après.

Fabrication du second module

Le second module se présente sous la forme d'une membrane, par exemple de polyuréthane, de perméabilité donnée (voir ci-après) à laquelle est fixé un copolymère hypoallergénique par exemple de type acrylique.

Les propriétés de ce copolymère et le grammage utilisé permettent d'optimiser ses propriétés d'adhésion à la peau.

Le second module est représenté par un schéma sur la figure 2.

Dans ce second module, l'épaisseur de la membrane de polyuréthane est d'environ 20 µm et celle de l'interface adhésive est comprise entre 30 et 40 µm.

Le procédé de fabrication de ce second module est globalement proche de celui du premier module. Néanmoins, certains paramètres : grammage, gradient de température sont différents.

Le film de polyuréthane utilisé dans ce second mobule joue un rôle capital dans le fonctionnement du système.

Sa perméabilité aux gaz et à la vapeur d'eau, donc sa composition et son épaisseur sont choisies afin d'optimiser le transfert de nitroglycérine du réservoir vers l'interface adhésive après contre-collage des deux modules.

De préférence, la perméabilité à la vapeur d'eau de la membrane est comprise entre 800 et 1.000 g/m²/24 h dans les conditions suivantes :

40°C ± 5°C, 82 ± 3 % HR.

Dans les mêmes conditions, la perméabilité à la vapeur d'eau de l'ensemble : membrane + interface adhésive est de préférence voisine de 400 g/m²/24 h.

Le protecteur anti-adhérent peut être constitué de tout complexe rigide ou semi-rigide anti-adhésif tels que PVC siliconé, polyester siliconé, polyester fluoré, polyester aluminisé siliconé, papier/aluminium/polyéthylène siliconé, ou tout autre composant adéquat.

Ce protecteur peut être directement enduit mais est, de préférence, collé après enduction d'un papier de protection temporaire.

En variante, on utilisera une interface préalablement chargée en nitroglycérine, la concentration en principe actif pouvant atteindre la saturation.

## Contrecollage des premier et second modules

Le papier de protection temporaire du premier module est retiré de manière à ce que le réservoir adhésif actif soit pressé par l'intermédiaire d'un cylindre presseur sur le film polyuréthane du second module. Ce contrecollage donne alors la structure représentée sur la figure 3a.

Ce dispositif est alors bobiné sous forme de rouleaux qui sont stockés pour maturation.

Lors de la maturation, la configuration du dispositif se modifie par transfert de nitroglycérine du réservoir adhésif actif vers l'interface adhésive, ce phénomène ne se produisant que dans le cas où l'on utilise une interface adhésive non saturée en principe actif.

En fin de maturation, le dispositif présente la configuration schématisée sur la figure 3b.

## Fendage des bobines et découpage

L'opération de fendage est destinée à faciliter l'opération de découpe. Elle consiste à réduire la laize des bobines du troisième module.

Le découpage est réalisé à l'aide d'un outil de découpe adéquat (emporte-pièce ou découpe rotative avec échenillage).

La surface découpée par exemple 5, 10, 20 ou 30 cm² permet de caractériser chaque dosage.

A titre d'illustration, on décrit la composition qualitative et quantitative d'un dispositif transdermique multilamellaire de surface unitaire de 20 cm² à base de résine acrylique.

```
- Support thermoplastique
  EVA/PVDC/EVA type CRYOVAC MF 200          20 cm²
- Copolymère acrylique (réservoir adhésif)  140 mg
  comportant de la nitroglycérine            50 mg
  Soit, copolymère acrylique actif
  (réservoir adhésif actif)                 190 mg
- Membrane polyuréthane                      20 cm²
- Copolymère acrylique (interface adhésive)  60 mg
- Protecteur polyester aluminisé siliconé*   20 cm²
```

## Fonctionnement du dispositif transdermique multilamellaire et résultats obtenus

### Fonctionnement

La nitroglycérine qui a migré du réservoir adhésif actif vers l'interface adhésive au travers de la membrane de polyuréthane a créé une interface activée en nitroglycérine. La saturation de cette interface est rapide puisqu'un temps de stockage de 24 heures suffit à l'établissement des conditions stationnaires de libération in vitro.

### Validation du profil in vitro

Ce profil in vitro (figure 4) réalisé 5 mois après la fabrication ne diffère pas significativement du profil réalisé à To soit 24 h après le contre-collage. Ceci confirme l'aptitude remarquable de la nitroglycérine à migrer au travers de cette membrane de polyuréthane et la stabilité du dispositif de la présente invention.

Par ailleurs, cette membrane joue un rôle dans le contrôle de la libération de la nitroglycérine à partir du réservoir adhésif actif comme le montre les profils in vitro (figure 5) réalisés sans membrane (à partir du premier module et avec membrane (à partir du dispositif multilamellaire).

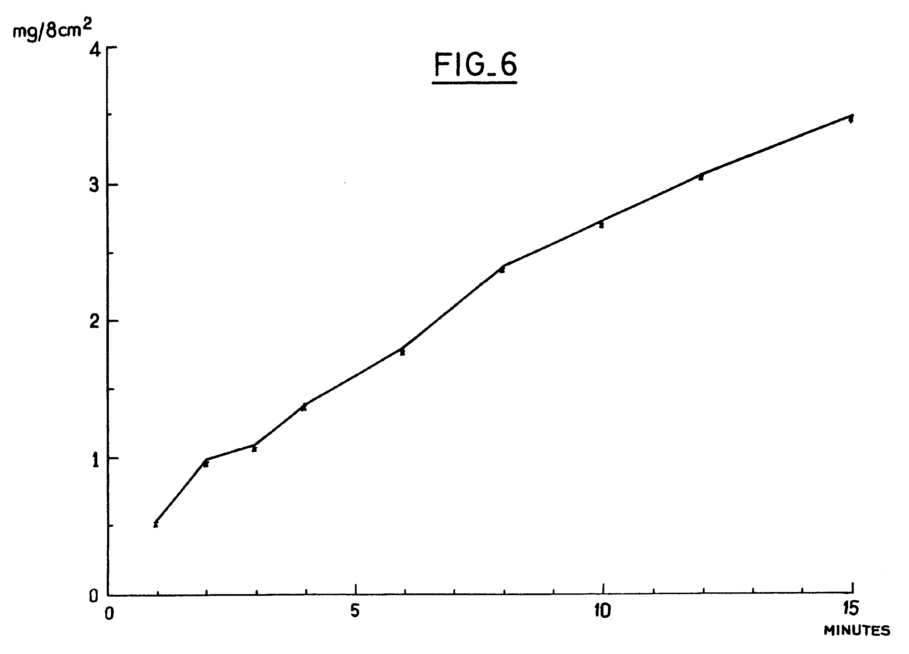

Au surplus, le suivi de la libération de la nitroglycérine sur les 15 premières minutes à partir du dispositif de la présente invention montre bien l'absence d'un phénomène flash (figure 6).

Validation in vivo : evaluation du profil plasmatique

La saturation en nitroglycérine de l'interface adhésive doit permettre d'obtenir une montée progressive des taux plasmatiques en deux heures environ et l'obtention d'un taux stable de nitroglycérine à l'état d'équilibre dès que le gradient de concentrations est établi au niveau des structures cutanées.

Le pré-essai pharmacocinétique réalisé sur trois sujets confirme cette hypothèse comme le montre la figure 7.

L'état d'équilibre est atteint environ deux heures après l'application du dispositif et les taux plasmatiques se maintiennent jusqu'à la 24ème heure ; le retrait du dispositif se traduit par une chute des taux plasmatiques.

## Revendications

1. Dispositif thérapeutique multilamellaire pour l'administration transdermique de nitroglycérine comportant successivement :

a) un support réalisé en un matériau souple et occlusif,

b) un réservoir adhésif actif à base de polymères de type acrylique ou silicone, incorporant le principe actif,

c) une membrane de transfert du principe actif, de type polyuréthane hautement perméable à la vapeur d'eau, présentant une structure microporeuse ou continue,

d) une interface adhésive hypoallergénique auto-alimentée par ledit réservoir en principe actif,

e) un protecteur réalisé en un film rigide ou semi-rigide, revêtu d'un anti-adhésif,

caractérisé en ce qu'il est constitué à partir d'un premier module comprenant le support, le réservoir adhésif actif et un papier de protection temporaire et un deuxième module comportant la membrane de transfert, l'interface adhésive et le protecteur anti-adhérent, les deux modules étant collés l'un sur l'autre après l'enlèvement du papier de protection du premier module par pressage du réservoir adhésif actif contre la membrane perméable du transfert du principe actif.

2. Dispositif selon la revendication 1, caractérisé en ce que le support est réalisé en un film thermoplastique coextrudé type EVA/PVDC/EVA.

3. Dispositif selon l'une quelconque des revendications 1 et 2, caractérisé en ce que le support est préalablement traité pour favoriser la fixation du réservoir adhésif actif.

4. Dispositif selon la revendication 1, caractérisé en ce que le grammage du principe actif dans le réservoir adhésif actif est compris entre 0,5 et 10 mg/cm$^2$.

5. Dispositif selon la revendication 1, caractérisé en ce que le rapport principe actif/polymères de type acrylique ou silicone est compris entre 0,1 et 0,6.

6. Dispositif selon l'une quelconque des revendications 1 à 5, caractérisé en ce que l'interface adhésive est à base de polymères de type acrylique ou silicone.

7. Dispositif selon la revendication 6, caractérisé en ce que le grammage des dérivés constitutifs de l'interface adhésive est compris entre 20 et 60 g/m$^2$, de préférence entre 30 et 40 g/m$^2$.

8. Dispositif selon l'une quelconque des revendications 1 à 7, caractérisé en ce qu'un antiseptique et/ou un adjuvant de perméation cutanée est incorporé dans le réservoir adhésif actif et/ou dans l'interface adhésive.

9. Dispositif selon l'une quelconque des revendications 1 à 8, caractérisé en ce que le film protecteur est réalisé en un film choisi parmi les papiers simples ou aluminisés, les polyesters ou les PVC.

10. Dispositif selon la revendication 9, caractérisé en ce que le film est revêtu d'un anti-adhésif choisi parmi les dérivés siliconés ou les dérivés fluorés.

11. Dispositif selon l'une quelconque des revendications 1 à 10, caractérisé en ce que l'épaisseur du support est compris entre 50 et 200 μm, l'épaisseur du réservoir adhésif actif est comprise entre 50 et 150 μm, l'épaisseur de la membrane est comprise entre 10 et 50 μm, l'épaisseur de l'interface adhésive est comprise entre 20 et 60 μm et l'épaisseur du protecteur est comprise entre 50 et 100 μm.

12. Dispositif selon la revendication 11, caractérisé en ce que l'épaisseur de l'interface adhésive est comprise entre 30 et 40 μm.

13. Procédé de préparation du dispositif selon l'une quelconque des revendications 1 à 12, caractérisé en ce qu'il comporte les étapes consistant à :

a) préparer un premier module comportant successivement :

. le support

. le réservoir adhésif actif,

. un papier de protection temporaire,

b) préparer un second module comportant successivement :

. la membrane de transfert du principe actif,

. l'interface adhésive,

. le protecteur anti-adhérent,

c) retirer le papier de transfert du premier module puis contre-coller les premier et second modules par pressage du réservoir adhésif actif du premier mobule contre la membrane perméable au principe actif du second module, et éventuellement

d) laisser reposer les modules contre-collés pour permettre à l'interface adhésive de se saturer progressivement en principe actif.

14. Procédé selon la revendication 13, caractérisé en ce que l'on utilise une interface adhésive préalablement chargée en principe actif.

15. Procédé selon la revendication 13, caractérisé en ce que l'étape d) est réalisée après avoir bobiné les modules contre-collés sous forme de rouleaux en vue du stockage.


## Patentansprüche

1. Multilamellare therapeutische Vorrichtung für die transdermische Verabreichung von Nitroglyzerin, die nacheinander folgendes umfaßt:

a) eine Halterung aus einem elastischen und verschliessendem Material,

b) einen klebenden aktiven Behälter auf der Basis eines Polymeren des Typs Acryl oder Silikon, in dem das aktive -Prinzip enthalten ist,

c) eine Transfermembrane für das aktive Prinzip vom Typ Polyurethan, das hoch durchlässig für Wasserdampf ist und das eine mikroporöse oder kontinuierliche Struktur aufweist,

d) eine hypoallergenische klebende Schnittstelle, die von dem Behälter automatisch mit dem aktiven Prinzip versorgt wird,

e) eine Schutzvorrichtung aus einer steifen oder halbsteifen Folie, die mit einem Antiklebemittel überzogen ist,

**dadurch gekennzeichnet, daß** sie aus einem ersten Modul gebildet wird, das die Halterung, den klebenden aktiven Behälter und ein temporäres Schutzpapier aufweist, sowie aus einem zweiten Modul, das die Transfermembrane, die klebende Schnittstelle und die nicht klebende Schutzvorrichtung aufweist, wobei die beiden Module nach Entfernung des Schutzpaiers des ersten Moduls durch Verpressung des klebenden aktiven Behälters mit der durchlässigen Membrane für den Transfer des aktiven Prinzips miteinander verklebt werden.

2. Vorrichtung nach Anspruch 1, **dadurch gekennzeichnet, daß** die Halterung aus einer gemeinsam verpressten thermoplastischen Folie vom Typ EVA/PVDC/EVA hergestellt ist.

3. Vorrichtung nach einem der Ansprüche 1 oder 2, **dadurch gekennzeichnet, daß** die Halterung vorbehandelt wird, um die Befestigung des klebenden aktiven Behälters zu vereinfachen.

4. Vorrichtung nach Anspruch 1, **dadurch gekennzeichnet, daß** die Flächenmasse des aktiven Prinzips in dem klebenden aktiven Behälter zwischen 0,5 und 10 mg/cm$^2$ beträgt.

5. Vorrichtung nach Anspruch 1, **dadurch gekennzeichnet, daß** das Verhältnis von aktivem Prinzip/Polymer vom Typ Acryl oder Silikon zwischen 0,1 und 0,6 beträgt.

6. Vorrichtung nach einem der Ansprüche 1 bis 5, **dadurch gekennzeichnet, daß** die klebende Schnittstelle auf der Basis eines Polymeren vom Typ Acryl oder Silikon hergestellt ist.

7. Vorrichtung nach Anspruch 6, **dadurch gekennzeichnet, daß** die Flächenmasse der die klebende Schnittstelle bildenden Derivate zwischen 20 und 60 g/m$^2$, vorzugsweise zwischen 30 und 40 g/m$^2$ beträgt.

8. Vorrichtung nach einem der Ansprüche 1 bis 7, **dadurch gekennzeichnet, daß** ein Antiseptikum und-/oder eine die kutane Eindringfähigkeit förderndes Adjuvans in den haftenden aktiven Behälter und/oder in die Klebende Schnittstelle integriert wird.

9. Vorrichtung nach einem der Ansprüche 1 bis 8, **dadurch gekennzeichnet, daß** der Schutzfilm aus einer Folie hergestellt ist, die aus einfachem oder aluminiertem Papier, aus Polyester oder PVC hergestellt wird.

10. Vorrichtung nach Anspruch 9, **dadurch gekennzeichnet, daß** die Folie mit einem Antihaftmittel überzogen ist, das aus Silikonderivaten oder Fluorderivaten hergestellt wird.

11. Vorrichtung nach einem der Ansprüche 1 bis 10, **dadurch gekennzeichnet, daß** die Wandstärke der Halterung zwischen 50 und 200 μm, die Wandstärke des klebenden aktiven Behälters zwischen 50 und 150 μm, die Wandstärke der Membrane zwischen 10 und 50 μm, die Wandstärke der klebenden Schnittstelle zwischen 20 und 60 μm und die Wandstärke der Schutzvorrichtung zwischen 50 und 100 μm beträgt.

12. Vorrichtung nach Anspruch 11, **dadurch gekennzeichnet, daß** die Wandstärke der klebenden Schnittstelle zwischen 30 und 40 μm beträgt.

13. Verfahren für die Herstellung der Vorrichtung nach einem der Ansprüche 1 bis 12, das durch folgende Fertigungsschritte gekennzeichnet ist:

a) Anfertigung eines ersten Moduls, das nacheinander folgendes aufweist:

. die Halterung,

. den klebenden aktiven Behälter,

. ein temporäres Schutzpapier,

b) Anfertigung eines zweiten Moduls, das nacheinander folgendes aufweist:

. die Membrane für den Transfer des aktiven Prinzips,

. die klebende Schnittstelle

. die nicht haftende Schutzvorrichtung

c) Abziehen des Transferpapiers des ersten Moduls, dann Verklebung des ersten und zweiten Moduls durch Verpressen des klebenden aktiven Behälters mit der für das aktive Prinzip des zweiten Moduls durchlässigen Membrane, und eventuell

d) Ruhenlassen der verklebten Module, um es der klebenden Schnittstelle zu ermöglichen, sich progressiv mit dem aktiven Prinzip vollzusaugen.

14. Verfahren nach Anspruch 13, **dadurch gekennzeichnet, daß** eine klebende Schnittstelle eingesetzt wird, die vorher mit dem aktiven Prinzip beladen wurde.

15. Verfahren nach Anspruch 13, **dadurch gekennzeichnet, daß** der Verfahrensschritt d) durchgeführt wird, nachdem die verklebten Module für die Lagerung in Form von Rollen aufgewickelt wurden.

## Claims

1. Multilamellar therapeutic device for the transdermal administration of nitroglycerin, comprising successively:

a) a support formed of a flexible and occlusive material,

b) an active adhesive reservoir based on polymers of the acrylic or silicone type, incorporating the active principle,

c) a membrane for the transfer of the active principle, of the polyurethane type highly permeable to water vapor, having a microporous or continuous structure,

d) a hypo-allergenic adhesive interface becoming self-supplied by said reservoir with active principle,

e) a protector formed of a rigid or semi-rigid film, coated with an anti-adhesive,

characterized in that it is formed from a first module comprising the support, the active adhesive reservoir and a temporary protection paper, and from a second module comprising the membrane for transfer, the adhesive interface and the anti-adhesive protector, the two modules being sticked one over the other, after removing the protection paper of the first module, by pressing the active adhesive reservoir against the permeable membrane for transfer of the active principle.

2. Device according to Claim 1, characterized in that the support is formed of a coextruded type EVA-/PbDC/EbA thermoplastic film.

3. Device according to any one of Claims 1 and 2, characterized in that the support is previously treated to facilitate the fixation of the active adhesive reservoir.

4. Device according to Claim 1, characterized in that the weight in grams per unit area of the active principle in the active adhesive reservoir is comprised between 0. 5 and 10 mg/cm$^2$.

5. Device according to Claim 1, characterized in that the ratio active principle/polymer of acrylic or silicone type is comprised between 0.1 and 0.6.

6. Device according to any one of Claims 1 to 5, characterized in that the adhesive interface is based on acrylic or silicone type polymers.

7. Device according to Claim 6, characterized in that the weight in grams per unit area of the constituent derivatives of the adhesive interface is comprised between 20 and 60 g/m$^2$, preferably between 30 and 40 g/m$^2$.

8. Device according to any one of Claims 1 to 7, characterized in that an antiseptic and/or an adjuvant for cutaneous permeation is incorporated in the active adhesive reservoir and/or in the adhesive interface.

9. Device according to any one of Claims 1 to 8, characterized in that the protector film is formed of a film selected from among simple or aluminized papers, polyesters or PVCs.

10. Device according to Claim 9, characterized in that the film is coated with an anti-adhesive agent selected from among silicone derivatives or fluorine derivatives.

11. Device according to any one of Claims 1 to 10, characterized in that the thickness of the support is comprised between 50 and 200 μm, the thickness of the active adhesive reservoir is comprised between 11 and 150 μm, the thickness of the membrane is comprised between 10 and 50 μm, the thickness of the adhesive interface is comprised between 20 and 60 μm and the thickness of the protector is comprised between 50 and 500 μm.

12. Device according to Claim 11, characterized in that the thickness of the adhesive interface is comprised between 30 and 40 μm.

13. Method for preparing the device according to any one of Claims 1 to 12, characterized in that it comprises the steps consisting of:

a) preparing a first module including successively:
. the support,
. the active adhesive reservoir,
. a temporary protector paper,

b) preparing a second module comprising successively:
. the membrane for transfer of the active principle,
. the adhesive interface,
. the anti-adhesive protector,

c) withdrawing the transfer paper from the first module and then back-sticking the first and second modules by pressing the active adhesive reservoir of the first module against the membrane permeable to the active principle of the second module, and possibly

d) leaving the back-stuck modules to stand to enable the adhesive interface to become gradually saturated with active principle.

14. Method according to Claim 13, characterized in that an adhesive interface is used previously charged with active principle.

15. Method according to Claim 13, characterized in that step d) is performed after having wound the back-stuck modules into roll form for storage.

FIG_1

FIG_2

FIG_3a

FIG_3b

FIG.4

mg/8cm²

HEURES

EP 0 318 385 B1

FIG.5

FIG. 6

mg/8cm²

EP 0 318 385 B1

14

MINUTES

CONCENTRATION (pg/ml)

FIG.7

TEMPS (heures)

EP 0 318 385 B1